# EUROPEAN PATENT SPECIFICATION

(11) **EP 4 583 965 B1**
(45) Date of publication and mention of the grant of the patent: **20.05.2026**
(21) Application number: 23741426.3
(22) Date of filing: 19.07.2023
(51) Int. Cl.: A61N 1/36, A61N 1/378, H02J 7/00

(54) **PSEUDO CONSTANT VOLTAGE CHARGING OF IMPLANTED MEDICAL DEVICE**
LADUNG EINER IMPLANTIERTEN MEDIZINISCHEN VORRICHTUNG MIT PSEUDOKONSTANTSPANNUNG
CHARGE À PSEUDO-TENSION CONSTANTE D'UN DISPOSITIF MÉDICAL IMPLANTÉ

(30) Priority: 07.09.2022 US 202263404431 P; 27.10.2022 EP 22204017
(43) Date of publication of application: 16.07.2025
(73) Proprietor: BIOTRONIK SE & Co. KG, 12359 Berlin (DE)
(72) Inventor: MCINTOSH, David, Wilsonville, Oregon 97070 (US); POEHLIG, Kai-Oliver, West Linn, Oregon 97068 (US); FRYER, Alan, Portland, Oregon 97202 (US)
(74) Representative: Biotronik Corporate Services SE
(86) International application number: PCT/EP2023/070040
(87) International publication number: WO 2024/051995

(56) References cited:
- EP-A1- 3 316 446
- CN-A- 105 162 181
- US-A- 5 367 244
- US-A- 6 087 810
- US-A1- 2008 269 724
- US-A1- 2011 279 079
- US-A1- 2015 196 768
- LEE EDWARD K F: "A Power Efficient LDO-Type Wireless Battery Charger for Biomedical Implants Based on Direct Charging from Regulated Rectifier Current", 2018 IEEE INTERNATIONAL SYMPOSIUM ON CIRCUITS AND SYSTEMS (ISCAS), IEEE, 27 May 2018 (2018-05-27), pages 1 - 4, XP033434399, DOI: 10.1109/ISCAS.2018.8350951

## Description

The present invention generally relates to a method for charging a battery of an implantable medical device. The present invention is further directed to a charging system comprising means for performing such a method, to an implantable medical device and a corresponding computer program.

A medical device, such as an implantable medical device (hereinafter referred to as IMD), is a useful tool as it contributes to a patient's health. Examples of IMDs include in a non-exhaustive list pacemakers, defibrillators, pressure sensors, neurostimulators, or the like. Thus, IMDs enjoy a wide range of applications. Furthermore, a demand for IMDs is expected to increase significantly as the value of IMDs is expected to become even more important in the future. Such an increased demand may be fueled by new medical technologies evolving that enable new potentials for IMDs, and this may include medical technologies not deemed realistic as from today's viewpoint.

A medical device, such as an implantable medical device, usually comprises a rechargeable battery, such as a lithium-ion battery (cell). A charging cycle (also referred to as recharging cycle) of the battery usually comprises two phases. First, a phase characterized by a constant current is applied, followed by a phase characterized by a constant voltage. In the constant current phase, a constant current (it may also be referred to as I_{cc}) is forced into the battery until the battery's voltage or the voltage of the battery's terminals reaches an end of charge voltage (ECV). In the constant voltage phase (also referred to as top-off phase), a constant voltage, which usually equals the ECV, is forced on the battery or the battery's terminals and the recharging current usually decays from the value in the constant current phase (I_{cc}) to a lower value, such as a cutoff value (I_{cutoff}) in an exponential manner.

The battery capacity is generally related to the ECV. Usually, lower ECV values lead to a reduced capacity. Furthermore, the ECV is related to the longevity, durability, lifecycle and/or lifespan (hereinafter the three terms may be referred to as longevity) of the battery. Typically, high values of the ECV result in a reduced longevity of the battery. Thus, a lower ECV value results in reduced capacity but increased longevity of the battery.

Nowadays, consumer devices, such as cell phones, are generally designed for 300 to 500 recharging cycles, which may provide for a device's lifecycle of about 1 to 2 years. On the other hand, IMDs generally may have a longer lifespan, for 5 years, 10 years or more, for instance for spinal cord stimulators.

As the series impedance of a battery's cell typically increases at increased battery age, the constant current phase typically becomes less effective in recharging the battery's cell. Hence, the constant voltage phase becomes more important for charging aged batteries. As an example, an aged cell having been in service for about 10 years may require 50% of its charging cycle to be in the constant voltage phase.

Many conventional consumer charging application-specific integrated circuits (hereinafter referred to as ASICs) may have fixed and relatively high ECV values of 4.2 V which renders them unsuitable for the application in IMDs. A limited number of ASICs with high levels of programmability allow for variable ECV values. Still, these programmable ASICs are far from being optimal. Moreover, they are generally not suited for IMDs, which require low power consumption.

Another drawback is that conventional integrated circuits (hereinafter referred to as ICs) used to control a charging process typically perform a trade-off between the following two opposing goals. The first goal is associated with an increased early charge rate which is critical for overall charge time, and high ECV values may be desired for that end. The second goal is associated with how much capacity is restored over the lifecycle of the battery and, to this end, low ECV values may be desired. Such a trade-off is detrimental as it usually does not lead to an overall optimal result. This is exacerbated by the fact that the efficacy of the constant current charging phase decreases over time.

US 2015/0196768 A1 describes a charging circuitry for receiving a magnetic charging field and using the received field to charge a battery in an Implantable Medical Device (IMD) without passive trickle charging, and even if the battery voltage (Vbat) is severely depleted. The charging circuitry includes a source capable of producing a constant charging current via a current mirror that receives a reference current for setting the charging current. Two reference current generators are provided: a first enabled when Vbat is severely depleted to produce a small non-adjustable reference current; and a second enabled once Vbat is recovered to produce a reference current that can be controlled to adjust the charging current. Because Vbat may be too low, the first generator is powered by a DC voltage produced from the magnetic charging field.

EP 331 644 6 A1 teaches a battery charging method that includes: charging a battery with a charging current; and changing the charging current in response to a current change event occurring during the charging of the battery, wherein the current change event occurs when the battery reaches a threshold voltage at which an anode potential of the battery reaches a reference value.

CN 105 162 181 A discloses a charging method and device. The method comprises the steps: judging whether a battery enters a constant-current charging state or not; setting the charging current of the battery according to a current voltage value of the battery if the battery enters the constant-current charging stage; carrying out the constant-current charging of the battery through the charging current; judging whether the voltage value of the battery after constant-current charging reaches a voltage value corresponding to the constant-current charging stage or not; and continuously executing the setting steps of the charging current according to the current voltage value of the battery if the voltage value of the battery after constant-current charging does not reach the voltage value corresponding to the constant-current charging stage.

US 5367244 A describes a method and apparatus to charge a battery including a DC charge current supply having a variable output. The charging current is varied in accordance with several sensed parameters in the circuit so that battery voltage is accurately controlled. Initially, constant charging current is applied, and upon detecting that battery voltage increases to the gassing voltage, an incremental step reduction in charging current is triggered. The step reduction causes a decrease in battery voltage, dropping it below the gassing voltage. The step reduced charging current is then applied to increase battery voltage back up to the gassing voltage, thereby triggering another step reduction in charging current. This process is repeated multiple times providing a stepped current profile, i.e., each battery voltage increase to the gassing voltage triggering a step reduction in charging current, and in turn a corresponding voltage reduction. The battery voltage, with the alternating increases and decreases, is thus defined by a saw-tooth profile with peaks at the gassing voltage. When the charging current is finally reduced to a minimum level, the sensed battery voltage triggers a termination of the stepped current reduction. Upon the battery reaching full charge, the application of charging current is terminated.

In view of the foregoing, there is a need to improve the known approaches for charging of IMDs. The aspects describe herein address the above need at least in part.

A first aspect relates to a method for charging a battery of an implantable medical device, IMD, as disclosed in claim 1.

Charging the battery (again) with a second substantially constant charging current (which may typically be lower than the first substantially constant charging current) aids in increasing and/or maximizing capacity of the battery being charged. The inventors found out that this advantage may be attributed to replacing the conventionally applied constant voltage phase by a (second) constant current phase. Instead of using a constant voltage and an exponentially decaying charging current, the battery may be repeatedly charged with several (different) constant charging currents. This may increase the duration of the constant current charging without having to increase ECV, which increases longevity of the battery. Hence, an optimized solution may be found for quick and effective charging combined with high longevity.

This particularly applies in combination with a relatively low ECV value set to facilitate a longevity of the battery that may be suitable for the IMD. This could be for instance more than 5 years, preferably more than 8 years, preferably more than 10 years. For example, it is expected that an ECV is required for an IMD, such as an implantable neurostimulator for spinal cord stimulation, which may be less than about 4.00 V to reach a longevity of about 9 years.

In view of the foregoing, the aspect described herein may enable an optimal solution without a burden to trade-off opposing goals: In particular, the aspect may provide for a rapid charging, such as a rapid initial charging.

Charging may generally be understood as a process that increases an amount of energy (of an element to be charged), such as an electrical or chemical energy or any other type of energy. The energy may be stored in a battery or accumulator. The term accumulator may be used synonymously to the term battery herein. It is noted that an IMD may comprise such an element to be charged (referred to herein as battery).

A charging current may be understood as a current that is directed to said battery. A current may result according to Ohm's law. As an example, a current may depend on a voltage and a resistance (or impedance; if not expressly stated otherwise herein, the term resistance may also be understood as impedance). The current may be substantially proportional to a voltage. Furthermore, it may be substantially anti-proportional to a resistance, e.g. proportional to a reciprocal value of a resistance.

As used herein, the term "substantially constant" may be understood to include (unwanted) deviations of a rather small amount, e.g. caused by noise or circuit imperfections. As an example, the deviations may be relative deviations, based on a reference value. The deviations may for example be at most 5%, preferably at most 2%, more preferably at most 1%, most preferably at most 0.5% of a reference value. As a reference value, an averaged value of the current of an interval, such as an interval over time may be referred to. The interval may be the time spent during the (e.g., constant current) charging step.

It is appreciated that the first and/or second substantially constant charging current may be detectably different from a varying and/or decaying charging current. As an example of such a varying and/or decaying charging current, a charging current of the second charging phase of conventional methods and/or systems may be mentioned (CV phase).

In one example, the method may switch from the first to the (different) second substantially constant charging current based on one or more predefined conditions. Such predefined conditions may take into account any quantities and/or parameters of the overall charging method.

Monitoring may be understood in such a way that a voltage of the battery is determined, measured, or retrieved otherwise. Monitoring may additionally or alternatively also mean that the voltage is determined, measured, or retrieved over an interval, such as a time interval. Preferably, the voltage is monitored during the overall charging process.

This has the advantage that such a monitored voltage could be used to control the charging process.

The threshold voltage may be an ECV of the battery. As an example, the ECV could be said to be an upper threshold limit. In such a way, during charging of an (empty or not fully charged) battery, the battery's voltage usually increases, until it reaches the ECV, upon which the charging process switches to the second substantially constant charging current.

The method may further comprise charging the battery with a third, fourth, and/or n-th substantially constant charging current, wherein n is an integer greater than 4. The method may further comprise switching from the second to the third, from the third to the fourth, and/or from the fourth to the n-th substantially constant charging current, when the monitored voltage substantially equals a threshold voltage, preferably an upper threshold voltage.

With this embodiment, it is possible to provide for several substantially constant charging currents during the charging process, which could enhance charging of the battery. In particular, it allows to set arbitrary ECV values (e.g. low ECV values to increase longevity of the battery), while still being able to charge the battery to a maximum amount by way of the (step wise) substantially constant charging currents.

Switching from the second to the third (and so forth) substantially constant charging currents may be similar as described in the preceding embodiment. In particular, the switching may occur when the monitored voltage (again) substantially equals the threshold voltage.

The second substantially constant charging current is smaller than the first substantially constant charging current, by 10 to 22% of the first substantially constant charging current.

A smaller second substantially constant charging current compared to the first one has the advantage that the battery voltage drops. Thus, charging by way of a second substantially constant charging current may be performed without exceeding the threshold voltage. This may provide for an increased restoration of the battery's energy without compromising longevity.

In a most preferred embodiment, the second substantially constant charging current may be smaller by about 14 to 19% or by 15 or 18% compared to the first one.

The first substantially constant charging current is at least 20 mA and/or at most 250 mA (the term "mA" may be understood as the unit milliamps).

A high first substantially constant charging current may provide for a quick charge.

However, it may be possible that it adversely affects an aging process of the battery. A low first substantially constant charging current may provide for an increased capacity restoration and/or utilization. However, it may entail a slow charge. The current values (i.e. the values of the current(s)) outlined herein may allow an optimal balance.

The threshold voltage, preferably the upper threshold voltage, is an end of charge voltage, ECV, preferably selected from the range 3.7 V to 4.1 V, such as approximately 3.8 V, 3.9 V or 4.0 V. In some examples, the ECV may be lower than 4.2 V, lower than 4.1 V or lower than 4.0 V.

A comparatively low value of an ECV is beneficial as it increases longevity of the battery. Compared to the conventional methods and/or systems (e.g. ECV of about 4.2 V), the ECV according to the present invention can be set lower, without adverse effects.

According to an 8th embodiment, the method may further comprise adjusting the threshold voltage, preferably the upper threshold voltage.

The method may allow to adjust an ECV to the individual battery to be charged and also to a certain state of a battery. Such a state may vary over the lifecycle and the method may provide for full benefits over the overall lifetime. As an example, the ECV could be reduced for an aged battery. Thus, a potential further aging of the battery may be counteracted or at least mitigated. At the same time, charging may be beneficially performed by way of several substantially constant currents to restore a maximum amount of capacity. In order to further facilitate charging, it may be possible to adapt the (several) values of the currents according to the adjusted threshold and/or a potentially increased impedance of an aged battery. It may also be viable to automate such an adjusting of the threshold voltage. In one example, this could be performed according to specific cells of a battery that are aged differently and may thus have different internal impedances.

The method may further comprise ceasing charging the battery after the step of charging the battery with the second substantially constant charging current, at least in part based on any one of the substantially, preferably a last, constant charging currents reaching a predetermined lower threshold current.

Ceasing charging may be understood in such a way that charging the battery is terminated and/or ended. Ceasing charging the battery may typically occur after charging the battery with the second substantially constant charging current.

Preferably, ceasing charging is performed after the last of the two or more substantially constant charging current phases (e.g. the second, the third, fourth and/or n-th, wherein n is an integer greater than 4).

As an example, if one of the substantially constant charging currents is lower than a lower threshold current, ceasing charging may be performed.

The method may further comprise ceasing charging the battery after the step of charging the battery with the second substantially constant charging current, at least in part based on the monitored voltage being substantially equal to a threshold voltage.

In one example, the threshold voltage may be the threshold voltage of the 3rd embodiment: This threshold voltage, such as the ECV, may be used to perform switching between substantially constant charging currents. Accordingly, it may also be possible that ceasing depends on an ECV (e.g. a threshold voltage). For example, if the ECV is reached, a switching to a next (lower) constant current would have to be performed. However, if this next (lower) constant current (e.g. the third, fourth, or n-th) is lower or equal to a threshold voltage, charging may be ceased.

Charging the battery with the first substantially constant charging current lasts at least 2 seconds, preferably at least 5 seconds, more preferably at least 20 seconds, most preferably at least 1 minute. Optionally, charging the battery with the first substantially constant charging current lasts at most 2 hours, preferably at most 1 hours, more preferably at most 30 minutes, most preferably at most 20 minutes.

According to another embodiment, charging the battery with the second substantially constant charging current and/or any further substantially constant charging current lasts at least 1 seconds, preferably at least 2 seconds, more preferably at least 3 seconds, most preferably at least 4 seconds. Optionally, charging the battery with the second substantially constant charging current and/or any further substantially constant charging current lasts at most 30 seconds, preferably at most 20 seconds, more preferably at most 10 seconds, most preferably at most 5 seconds.

It may be appreciated that the time for charging with the first, second or any other substantially constant charging current may be independent from one another. This may facilitate flexibility. It may also be advantageous that the time for the second or any further substantially constant charging current is lower compared to the first one. This may increase restoration of capacity.

A second aspect relates to a charging system for charging a battery of an implantable medical device, IMD, as disclosed in claim 8.

Moreover, the charging system may comprise means for adjusting a threshold voltage, preferably an upper threshold voltage. Optionally, the charging system comprises means for switching to further substantially constant charging currents. It is noted that the means for switching from the first to a second substantially constant charging current may, in one example, be the same means as the means for switching to any further substantially constant charging current. In another example, such means may be different and/or separate means as the means for switching to any further substantially constant charging current. Substantially constant charging current values may, e.g., be stored, saved, established, or otherwise made available and/or accessible to be used by the charging system. It may also be possible that such currents are programmed in an integrated circuit.

As an example, the charging system may comprise a charging integrated circuit, IC. Such an IC may be connected to a battery for providing a current to the battery. The IC may be configured to charge the battery with a first substantially constant charging current and with a second substantially constant charging current.

Furthermore, the charging system may comprise a controller, which could be operatively connected to the IC. The controller could be a microcontroller. The controller may have stored one or more values of substantially constant charging currents. In addition, the controller may be configured to monitor a voltage of the battery. It may be possible that the controller is able to monitor the voltage of the battery's terminals. In such a way, part of the controller may be referred to as the means for monitoring a voltage of the battery. Furthermore, the controller may be configured to perform switching from the first to the second substantially constant charging current, when the monitored voltage substantially equals a threshold voltage, preferably an upper threshold voltage.

Moreover, the controller may be configured to perform switching from the second to the third, from the third to the fourth, and/or from the fourth to the n-th substantially constant charging current, when the monitored voltage substantially equals a threshold voltage, preferably an upper threshold voltage.

The second substantially constant charging current is smaller than the first substantially constant charging current, by 10 to 22% of the first substantially constant charging current.

The first substantially constant charging current is at least 20 mA, and/or at most 250 mA.

It is noted that the charging system as described herein may include all aspects and/or embodiments described herein, even if not expressly described as being part of the charging system but rather with reference to a method. It is also to be understood that the features and advantages described with reference to the charging system may equally be applicable to the method steps. Moreover, the charging system as outlined herein may include means for implementing all aspects and/or embodiments as outlined herein, even if these may rather be described in the context of method steps. Furthermore, the features and advantages described with reference to the method steps may equally be applicable to the charging system.

A third aspect relates to an implantable medical device, IMD, comprising a charging system according to any one of aspects and/or embodiments described herein. The charging system and/or IMD may also comprise a housing.

The housing may also comprise a portion which is used to be coupled to one or more leads in order to deliver energy. The energy could be delivered to a stimulation system.

A fourth aspect of the invention is related to a computer program comprising instructions which, when the program is executed by a computer, cause the computer to carry out the steps of the methods described herein.

Whether described as method steps, computer program and/or means, the functions described herein may be implemented in hardware, software, firmware, and/or combinations thereof. If implemented in software/firmware, the functions may be stored on or transmitted as one or more instructions or code on a computer-readable medium.

Computer-readable media include both computer storage media and communication media including any medium that facilitates transfer of a computer program from one place to another. A storage medium may be any available media that can be accessed by a general purpose or special purpose computer. By way of example, and not limitation, such computer-readable storage media can comprise RAM, ROM, EEPROM, FPGA, CD/DVD or other optical disk storage, magnetic disk storage or other magnetic storage devices, or any other medium that can be used to carry or store desired program code means in the form of instructions or data structures and that can be accessed by a general-purpose or special-purpose computer, or a general-purpose or special-purpose processor.

The charging system as described herein may also be implemented in hardware, software, firmware, and/or combinations thereof, for example, by means of one or more general-purpose or special-purpose computers, and/or a general-purpose or special-purpose processors.

In the following, preferred embodiments are described, by way of example only. Reference is made to the following accompanying figures:
- Fig. 1: Schematic representation of two diagrams comprising a charging current and a battery voltage over time of a charging process according to conventional methods and/or systems.
- Fig. 2: Schematic representation of two diagrams comprising a charging current and a battery voltage over time of a charging process according to an exemplary embodiment of a method and/or charging system according to the present invention.
- Fig. 3: Schematic representation of a circuit block diagram that illustrates an example of a charging system according to the present invention.
- Fig. 4: Schematic representation of a flow diagram that illustrates an example of a method and/or charging system according to the present invention.
- Fig. 5: Schematic representation of a flow chart that illustrates an example of a method for charging a battery of an implantable medical device, IMD, according to the present invention.

In the subsequent passages, the invention is described with reference to the accompanying figures in more detail. It is noted that further embodiments are certainly possible, and the below explanations are provided by way of example only, without limitation.

While specific feature combinations are described with respect to the exemplary embodiments of the present invention, it is to be understood that not all features of the discussed embodiments have to be present for realizing the invention, which is defined by the subject matter of the claims. The disclosed embodiments may be modified by combining certain features of one embodiment with one or more features of another embodiment. Specifically, the skilled person will understand that features, components and/or functional elements of one embodiment can be combined with technically compatible features, components and/or functional elements of any other embodiment of the present invention given that the resulting combination falls within the definition of the invention provided by the claims. The skilled person also understands that certain features may be omitted in so far as they appear dispensable.

Throughout the present figures and specification, the same reference numerals refer to the same elements. The figures may not be to scale, and the relative size, proportions, and depiction of elements in the figures may be exaggerated for clarity, illustration, and convenience.

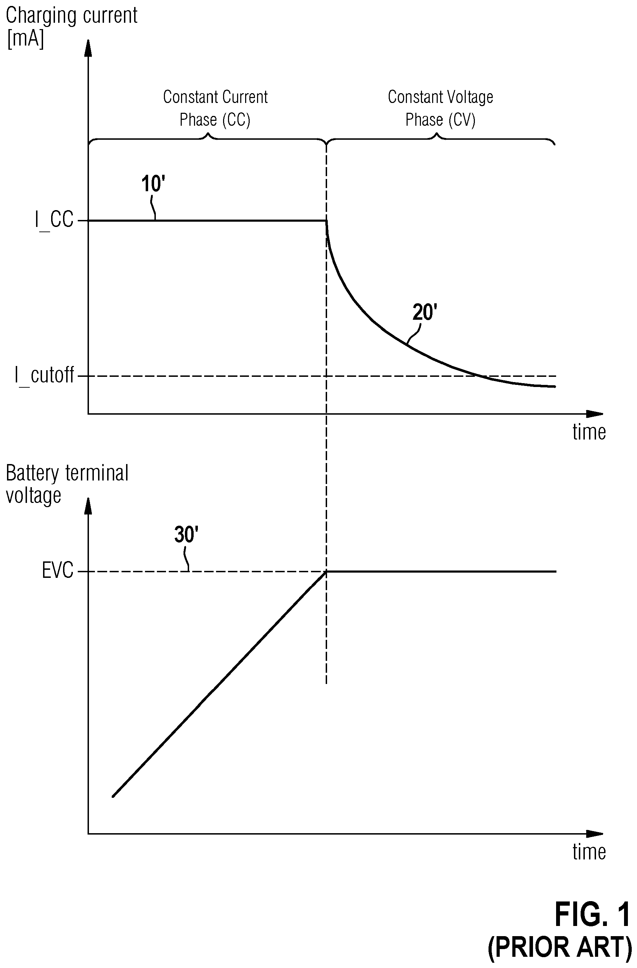

Fig. 1 shows a schematic representation of two diagrams comprising a charging current and a battery voltage over time of a charging process according to conventional methods and/or systems.

The upper diagram shows the charging current in mA over time. The lower diagram shows the battery voltage (which could be the battery terminal voltage) over time. The battery is charged in a first, constant current (CC) phase 10' and a second, second voltage (CV) phase 20'. In the first phase, a constant current is forced into the battery until the battery terminal voltage reaches an end of charge voltage, ECV 30'. In the second phase, the ECV 30' is maintained and the charging current decays smoothly until a cutoff current (I_{cutoff}) is reached.

In this example, the ECV could be set high, which increases capacity, but reduces longevity of the battery, which is a major drawback and renders such a battery unsuitable for an application in IMDs. As explained herein, when the battery ages, the second, CV phase 20' is more demanded, as the CV phase 10' is less effective in replenishing energy in the battery, e.g. the battery's cells. As an example, an aged battery's cell of 10 years, may require 50% of its charging process to be in the CV phase.

Such a fixed method and/or system of the prior art, requires to trade-off between an early charge rate, which is critical for overall charging time with how much capacity is restored over the life of the cell.

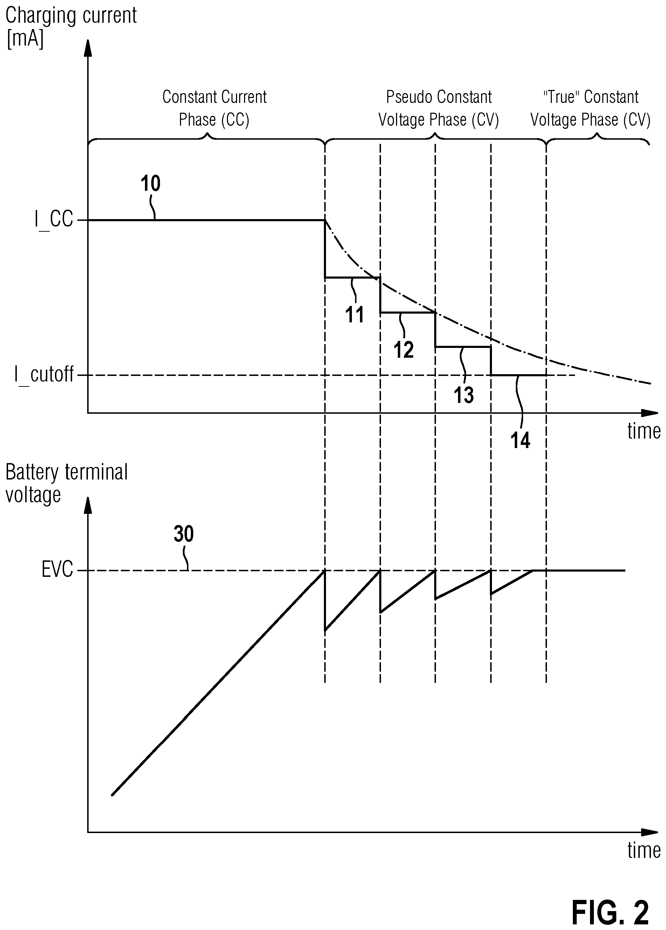

Fig. 2 shows a schematic representation of two diagrams showing a charging current and a battery voltage over time of a charging process according to an exemplary embodiment of a method and/or charging system according to the present invention. As an example, the battery to be charged could be a lithium-ion battery (cell) or any related suitable battery for an IMD.

The diagram's axes may be described similarly to the axes of the diagrams of the Fig. 1.

According to Fig. 2, the battery is charged with a first substantially constant charging current 10. Further, the battery may be charged with a second substantially constant charging current 11. Optionally, further substantially constant charging currents may follow, e.g. a third 12, a fourth 13, a fifth 14 (or an n-th charging current, wherein n is an integer greater than 4). It is noted that the second substantially constant charging current is lower compared to the first substantially constant charging current. If charging with further substantially constant charging currents is performed, the subsequent current may typically be smaller compared to the respective preceding one.

As shown in the lower diagram of Fig. 2, the terminal voltage drops, when the charging current is reduced. This may be attributable to Ohm's law, such that a reduced current leads to a reduced voltage (in case the resistance, e.g. the impedance is kept constant). An impedance is understood as the opposition to alternating current (AC) presented by the combined effect of resistance and reactance in a circuit. As an example, the resistance may be the corresponding term for direct current (DC). The conventional constant voltage phase may be approximated by a pseudo constant voltage phase, which may consist of several discrete steps, e.g. substantially constant charging currents.

Charging with a subsequent substantially constant charging current may be performed until a cutoff current (I_{cutoff}) is reached, e.g. a constant current is lower or equal to I_{cutoff}.

Thus, the schematic representation of Fig. 2 can provide for a rapid charging, e.g. a rapid initial charging, which minimizes overall charging time of the battery. Furthermore, the advantage of the CV phase may also be maintained (by way of one or more substantially constant charging currents) to recover as much capacity as possible for the battery's cells. This may particularly be the case, if the battery ages, which typically leads to an increase of a series impedance in the cells, as an increased series impedance reduces the time spent in the conventional constant current phase.

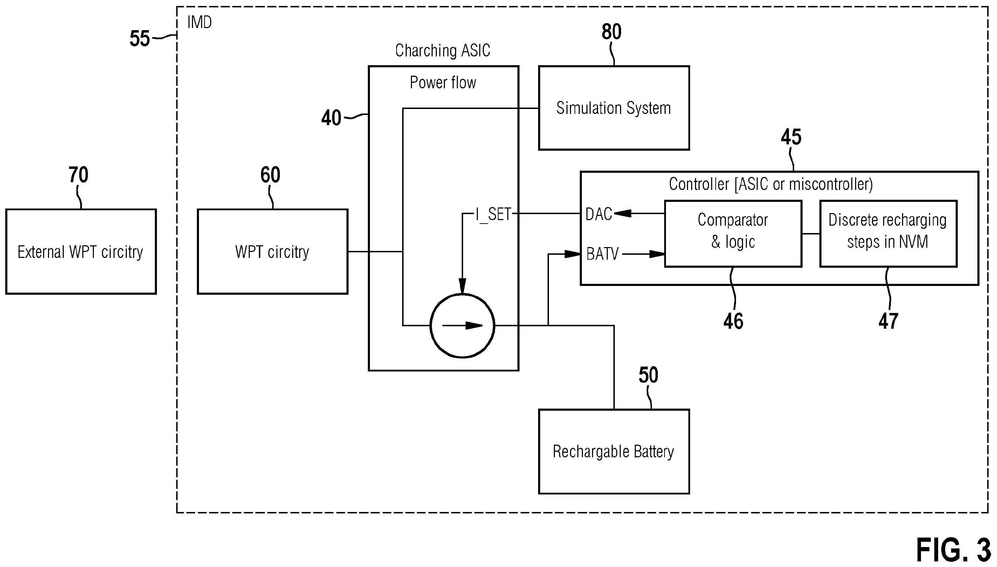

Fig. 3 shows a schematic representation of a circuit block diagram that illustrates an example of a charging system according to the present invention which is comprised by an IMD.

As can be seen, an IMD 55 is shown which may comprise a rechargeable battery 50. As an example, the IMD 55 may be a pacemaker, defibrillator, pressure sensor, neurostimulator, or the like. Rechargeable battery 50 of IMD 55 could be integral with the IMD 55. A wireless power transfer (WPT) coil and respective WPT circuitry 60 may be comprised by the IMD 55. Furthermore, a respective external WPT circuitry 70 may be provided to deliver energy to the IMD 55. The WPT circuitry 60 may comprise resonant capacitors, rectifiers, storage capacitors and/or over-voltage protection. Alternative wireless powering technologies suitable for applications in IMDs include powering based on ultrasound, capacitive coupling, radiofrequency, or optical means.

In addition, a charging integrated circuit (IC) 40, such as a charging application-specific integrated circuit (ASIC) may be comprised by the IMD 55 and used to recharge battery 50 from a link, such as a DC link voltage, which may be generated from the WPT coil.

The IMD 55 may further comprise a controller 45. As an example, the controller 45 could be an ASIC or a microcontroller. The controller 45 operatively interfaces, e.g. via a digital to analog converter (DAC) to the charging IC 40, e.g. via an input port of charging IC 40. The controller 45 may be able to set one or more substantially constant charging currents I_SET to the charging IC 40 and to the rechargeable battery 50.

As an example, one or more values of (discrete) substantially constant charging currents may be set within the controller 45. For instance, the values may be stored within the controller 45 or known otherwise. The values may comprise 90 mA, 75 mA, 60 mA, 45 mA, 30 mA and 15 mA. However, different values may also be possible, and the described figure is not limited to the above-mentioned values.

The controller 45 can monitor a battery voltage (BATV). The monitored voltage may be processed and/or compared to a threshold voltage, e.g. by a comparator and logic unit 46. Furthermore, the controller 45 may store a defined threshold voltage, e.g. ECV. As an example, the ECV could be stored in non-volatile memory. The controller 45 may comprise means for storing discrete recharging steps 47. Said means could be a non-volatile memory. In one example, the ECV could be stored in said means 47 as well. Alternatively, the discrete recharging steps may be stored where the ECV is stored. The discrete recharging steps may be the one or more values of (discrete) substantially constant charging currents as described herein.

The controller 45 may set a subsequent substantially constant charging current from the one or more values (e.g. 90 mA, 75 mA, 60 mA, 45 mA, 30 mA and 15 mA) to the charging IC 40, each time the ECV is reached. Typically, the BATV increases as the battery 50 is charged with a charging current. Thus, it may take some time until the ECV is reached. Such a procedure may also be termed a step down through discrete currents.

Charging with a subsequent substantially constant charging current may be performed until a last charging current from the one or more values is reached (e.g. 90 mA, 75 mA, 60 mA, 45 mA, 30 mA and 15 mA). However, charging at the last charging current may still be possible. In some examples, not all current values may be used. Instead, charging may be stopped when the next current value reaches a cutoff current (I_{cutoff}). Thus, reaching such a value may be accompanied with ceasing charging the battery 50.

Furthermore, as exemplarily indicated in Fig. 3, the IMD may comprise a stimulation system 80 which may be provided with energy by the rechargeable battery 50.

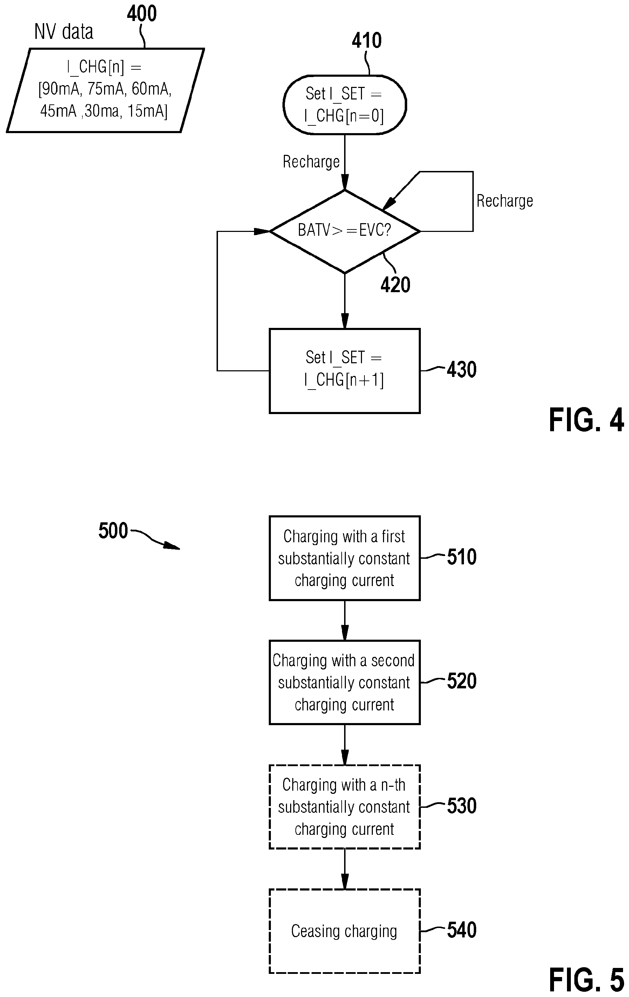

Fig. 4 shows a schematic representation of a flow diagram that illustrates an example of a method and/or charging system according to the present invention.

The flow diagram exemplarily describes the method and/or charging system according to the present invention.

One or more values of substantially constant charging currents may be saved and/or stored as NV (non-volatile) data 400. As an example, said one or more values may be stored in the controller 45 as described with reference to Fig. 3. Thereby, the controller 45 may have a (fixed) set of discrete charging currents in its memory. The corresponding values I_CHG[n] may comprise the values 90 mA, 75 mA, 60 mA, 45 mA, 30 mA and 15 mA. It is noted that different values are also be possible, the values may depend on the IMD and/or the battery.

A first substantially constant charging current may be set as I_SET in step 410, which is indicated as "Set I_SET = I_CHG[n=0]". Accordingly, the battery is charged. Subsequently, the BATV is monitored in step 420. Furthermore, the monitored BATV is compared with the ECV. If the BATV is equal to and/or greater than the ECV, a next subsequent substantially constant charging current may be set, which is indicated as "Set I_SET = I_CHG[n+1]" in step 430. The monitoring (of the BATV in step 420), comparing (whether BATV is equal to and/or greater than the ECV in step 420) and setting of a subsequent substantially constant charging current (n+1) in step 430 may be repeated.

It is understood, that in this example the term n is not limited to an integer greater than 4. For example, n could be an integer greater or equal to 0 as derivable from Fig. 4.

It is noted that, during the time of the exemplary flow diagram, charging of the battery is performed, indicated as "Recharge..." in this figure.

Typically, ceasing (not explicitly indicated in Fig. 4 for brevity only) charging the battery occurs during or after reaching the last value of one or more values of substantially constant charging currents.

Fig. 5 shows a schematic representation of a flow chart that illustrates an example of a method for charging a battery of an implantable medical device, IMD, according to the present invention.

The method 500 comprises the step of charging the battery with a first substantially constant charging current 510.

Furthermore, the method 500 comprises the step of charging the battery with a second substantially constant charging current 520.

Optionally (as indicated by the dashed box), the method 500 comprises the step of charging the battery with a third, fourth, and/or n-th substantially constant charging current 530, wherein n is an integer greater than 4.

Preferably (as indicated by the dashed box), the method 500 comprises the step of ceasing charging 540 the battery after the step of charging the battery with the second (third, fourth and/or n-th) substantially constant charging current. This may at least in part be based on any one of the substantially, preferably a last, constant charging currents reaching a predetermined lower threshold current (e.g. a I_{cutoff} value). Additionally or alternatively, it may at least in part be based on the monitored voltage being substantially equal to a threshold voltage (e.g. an ECV value).

### REFERENCE NUMERALS

- AC: alternating current
- ASIC: application-specific integrated circuit
- BATV: battery voltage
- DC: direct current
- IC: integrated circuit
- WPT: wireless power transfer

- 10': constant current phase of prior art
- 20': constant voltage phase of prior art
- 30': end of charge voltage, ECV, of prior art

- 10: first substantially constant current
- 11: second substantially constant current
- 12: third substantially constant current
- 13: fourth substantially constant current
- 14: fifth/n-th substantially constant current

- 30: end of charge voltage, ECV

- 40: charging IC
- 45: controller
- 46: comparator and logic unit
- 47: means for storing (e.g. non-volatile memory)
- 50: (rechargeable) battery

- 60: WPT circuitry
- 70: external WPT circuitry

- 55: implantable medical device, IMD

- 80: stimulation system

- 400: storing one or more values of substantially constant charging currents

- 410: setting a first substantially constant charging current
- 420: monitoring and checking a BATV
- 430: setting a subsequent substantially constant charging current

- 500: method for charging a battery
- 510: method step: charging with a first substantially constant charging current
- 520: method step: charging with a second substantially constant charging current
- 530: method step: charging with a n-th substantially constant charging current
- 540: method step: ceasing charging

## Claims

1. A method for charging a battery of an implantable medical device, IMD, the method comprising:
- charging the battery with a first substantially constant charging current;
- charging the battery with a second substantially constant charging current;
- monitoring a voltage of the battery;
**characterized in that**
- the method further comprises the step of switching from the first to the second substantially constant charging current, when the monitored voltage substantially equals a threshold voltage,
wherein the second substantially constant charging current is smaller than the first substantially constant charging current by 10 to 22% of the first substantially constant charging current,
and wherein the first substantially constant charging current is at least 20 mA and at most 250mA.

2. The method according to claim 1, further comprising:
charging the battery with a third, fourth, and/or n-th substantially constant charging current, wherein n is an integer greater than four;
switching from the second to the third, from the third to the fourth, and/or from the fourth to the n-th substantially constant charging current, when the monitored voltage substantially equals a threshold voltage, preferably an upper threshold voltage.

3. The method according any one of the preceding claims, wherein the threshold voltage is an end of charge voltage, ECV from the range 3.7 V to 4.1 V.

4. The method according to any one of the preceding claims, further comprising adjusting the upper threshold voltage.

5. The method according to any one of the preceding claims, further comprising:
ceasing charging the battery after the step of charging the battery with the second substantially constant charging current, at least in part based on any one of the substantially constant charging currents reaching a predetermined lower threshold current.

6. The method according to any one of the preceding claims, further comprising:
ceasing charging the battery after the step of charging the battery with the second substantially constant charging current, at least in part based on the monitored voltage being substantially equal to a threshold voltage.

7. The method according to any one of the preceding claims, wherein charging the battery with the first substantially constant charging current lasts at least 2 seconds; and/or
wherein charging the battery with the first substantially constant charging current lasts at most 2 hours; and/or
wherein charging the battery with the second substantially constant charging current and/or any further substantially constant charging current lasts at least 1 seconds; and/or
wherein charging the battery with the second substantially constant charging current and/or any further substantially constant charging current lasts at most 30 seconds.

8. A charging system for charging a battery of an implantable medical device, IMD, the charging system comprising
means for charging the battery with a first substantially constant charging current and for charging the battery with a second substantially constant charging current;
means for monitoring a voltage of the battery;
**characterized in that**
the charging system further comprises means for switching from the first to the second substantially constant charging current, when the monitored voltage substantially equals a threshold voltage,
wherein the second substantially constant charging current is smaller than the first substantially constant charging current by 10 to 22% of the first substantially constant charging current,
and wherein the first substantially constant charging current is at least 20 mA and at most 250mA.

9. The charging system according to claim 8, the charging system further comprises a charging integrated circuit (IC) and a controller operatively connected to the IC, wherein the controller is configured to
charge the battery with a third, fourth, and/or n-th substantially constant charging current, wherein n is an integer greater than four;
switch from the second to the third, from the third to the fourth, and/or from the fourth to the n-th substantially constant charging current, when the monitored voltage substantially equals a threshold voltage, preferably an upper threshold voltage.

10. An implantable medical device, IMD, comprising a charging system according to anyone of the claims 8 or 9.

11. A computer program comprising instructions which, when the program is executed by a computer, causes the computer to carry out the steps of the method of claims 1 to 7.

## Patentansprüche

1. Verfahren zum Laden einer Batterie eines implantierbaren medizinischen Geräts, IMD, wobei das Verfahren umfasst:
- Laden der Batterie mit einem ersten im Wesentlichen konstanten Ladestrom;
- Laden der Batterie mit einem zweiten im Wesentlichen konstanten Ladestrom;
- Überwachen einer Spannung der Batterie;
**dadurch gekennzeichnet, dass**
- das Verfahren ferner den Schritt des Umschaltens vom ersten zum zweiten im Wesentlichen konstanten Ladestrom umfasst, wenn die überwachte Spannung im Wesentlichen einer Schwellenspannung entspricht,
wobei der zweite im Wesentlichen konstante Ladestrom um 10 bis 22 % des ersten im Wesentlichen konstanten Ladestroms kleiner ist als der erste im Wesentlichen konstante Ladestrom,
und wobei der erste im Wesentlichen konstante Ladestrom wenigstens 20 mA und höchstens 250 mA beträgt.

2. Verfahren nach Anspruch 1, ferner umfassend:
Laden der Batterie mit einem dritten, vierten und/oder n-ten im Wesentlichen konstanten Ladestrom, wobei n eine ganze Zahl größer als vier ist;
Umschalten vom zweiten zum dritten, vom dritten zum vierten und/oder vom vierten zum n-ten im Wesentlichen konstanten Ladestrom, wenn die überwachte Spannung im Wesentlichen einer Schwellenspannung, vorzugsweise einer oberen Schwellenspannung, entspricht.

3. Verfahren nach einem der vorangehenden Ansprüche, wobei die Schwellenspannung eine Ladungsendspannung, ECV, aus dem Bereich von 3,7 V bis 4,1 V ist.

4. Verfahren nach einem der vorangehenden Ansprüche, ferner umfassend das Anpassen der oberen Schwellenspannung.

5. Verfahren nach einem der vorangehenden Ansprüche, ferner umfassend:
Beenden des Ladens der Batterie nach dem Schritt des Ladens der Batterie mit dem zweiten im Wesentlichen konstanten Ladestrom, wenigstens teilweise basierend darauf, dass einer der im Wesentlichen konstanten Ladeströme einen vorbestimmten unteren Schwellenstrom erreicht.

6. Verfahren nach einem der vorangehenden Ansprüche, ferner umfassend:
Beenden des Ladens der Batterie nach dem Schritt des Ladens der Batterie mit dem zweiten im Wesentlichen konstanten Ladestrom, wenigstens teilweise basierend darauf, dass die überwachte Spannung im Wesentlichen gleich einer Schwellenspannung ist.

7. Verfahren nach einem der vorangehenden Ansprüche, wobei das Laden der Batterie mit dem ersten im Wesentlichen konstanten Ladestrom wenigstens 2 Sekunden dauert; und/oder
wobei das Laden der Batterie mit dem ersten im Wesentlichen konstanten Ladestrom höchstens 2 Stunden dauert; und/oder
wobei das Laden der Batterie mit dem zweiten im Wesentlichen konstanten Ladestrom und/oder einem weiteren im Wesentlichen konstanten Ladestrom wenigstens 1 Sekunde dauert; und/oder
wobei das Laden der Batterie mit dem zweiten im Wesentlichen konstanten Ladestrom und/oder einem weiteren im Wesentlichen konstanten Ladestrom höchstens 30 Sekunden dauert.

8. Ladesystem zum Laden einer Batterie eines implantierbaren medizinischen Geräts, IMD, wobei das Ladesystem umfasst:
Mittel zum Laden der Batterie mit einem ersten im Wesentlichen konstanten Ladestrom und zum Laden der Batterie mit einem zweiten im Wesentlichen konstanten Ladestrom;
Mittel zum Überwachen einer Spannung der Batterie;
**dadurch gekennzeichnet, dass**
das Ladesystem ferner Mittel zum Umschalten vom ersten zum zweiten im Wesentlichen konstanten Ladestrom umfasst, wenn die überwachte Spannung im Wesentlichen einer Schwellenspannung entspricht,
wobei der zweite im Wesentlichen konstante Ladestrom um 10 bis 22 % des ersten im Wesentlichen konstanten Ladestroms kleiner ist als der erste im Wesentlichen konstante Ladestrom,
und wobei der erste im Wesentlichen konstante Ladestrom wenigstens 20 mA und höchstens 250 mA beträgt.

9. Ladesystem nach Anspruch 8, wobei das Ladesystem ferner eine integrierte Schaltung (IC) zum Laden und eine mit der IC funktionsfähig verbundene Steuerung umfasst, wobei die Steuerung dazu ausgestaltet ist,
die Batterie mit einem dritten, vierten und/oder n-ten im Wesentlichen konstanten Ladestrom zu laden, wobei n eine ganze Zahl größer als vier ist;
vom zweiten zum dritten, vom dritten zum vierten und/oder vom vierten zum n-ten im Wesentlichen konstanten Ladestrom umzuschalten, wenn die überwachte Spannung im Wesentlichen einer Schwellenspannung, vorzugsweise einer oberen Schwellenspannung, entspricht.

10. Implantierbares medizinisches Gerät, IMD, umfassend ein Ladesystem nach einem der Ansprüche 8 oder 9.

11. Computerprogramm mit Anweisungen, die, wenn das Programm von einem Computer ausgeführt wird, den Computer veranlassen, die Schritte des Verfahrens der Ansprüche 1 bis 7 auszuführen.

## Revendications

1. Procédé de charge d'une batterie d'un dispositif médical implantable, ou IMD, le procédé comprenant les étapes consistant à :
- charger la batterie avec un premier courant de charge essentiellement constant ;
- charger la batterie avec un deuxième courant de charge essentiellement constant ;
- suivre une tension de la batterie ;
**caractérisé en ce que**
- le procédé comprend en outre l'étape consistant à commuter du premier au deuxième courant de charge essentiellement constant, lorsque la tension suivie est essentiellement égale à une tension de seuil,
dans lequel le deuxième courant de charge essentiellement constant est inférieur au premier courant de charge essentiellement constant de 10 à 22 % du premier courant de charge essentiellement constant,
et dans lequel le premier courant de charge essentiellement constant est d'au moins 20 mA et d'au plus 250 mA.

2. Procédé selon la revendication 1, comprenant en outre les étapes consistant à :
charger la batterie avec un troisième, un quatrième et/ou un n-ième courant de charge essentiellement constant, dans lequel n représente un entier supérieur à quatre ;
commuter du deuxième au troisième, du troisième au quatrième et/ou du quatrième au n-ième courant de charge essentiellement constant, lorsque la tension suivie est essentiellement égale à une tension de seuil, de préférence une tension de seuil supérieur.

3. Procédé selon l'une quelconque des revendications précédentes, dans lequel la tension de seuil est une tension de fin de charge, ou ECV, dans la plage de 3,7 V à 4,1 V.

4. Procédé selon l'une quelconque des revendications précédentes, comprenant en outre l'étape consistant à ajuster la tension de seuil supérieur.

5. Procédé selon l'une quelconque des revendications précédentes, comprenant en outre l'étape consistant à :
interrompre la charge de la batterie après l'étape consistant à charger la batterie avec le deuxième courant de charge essentiellement constant, en se basant au moins en partie sur le fait que l'un quelconque des courants de charge essentiellement constants atteint un courant de seuil inférieur prédéterminé.

6. Procédé selon l'une quelconque des revendications précédentes, comprenant en outre l'étape consistant à :
interrompre la charge de la batterie après l'étape consistant à charger la batterie avec le deuxième courant de charge essentiellement constant, en se basant au moins en partie sur le fait que la tension suivie est essentiellement égale à une tension de seuil.

7. Procédé selon l'une quelconque des revendications précédentes, dans lequel l'étape consistant à charger la batterie avec le premier courant de charge essentiellement constant dure au moins 2 secondes ; et/ou
dans lequel l'étape consistant à charger la batterie avec le premier courant de charge essentiellement constant dure au maximum 2 heures ; et/ou
dans lequel l'étape consistant à charger la batterie avec le deuxième courant de charge essentiellement constante et/ou n'importe quel courant de charge essentiellement constant supplémentaire dure au moins 1 seconde ; et/ou
dans lequel l'étape consistant à charger la batterie avec le deuxième courant de charge essentiellement constant et/ou n'importe quel courant de charge essentiellement constant supplémentaire dure au plus 30 secondes.

8. Système de charge destiné à charger une batterie d'un dispositif médical implantable, ou IMD, le système de charge comprenant
un moyen de charge de la batterie avec un premier courant de charge essentiellement constant et de charge de la batterie avec un deuxième courant de charge essentiellement constant ;
un moyen de suivre une tension de la batterie ;
**caractérisé en ce que**
le système de charge comprend en outre un moyen de commutation du premier au deuxième courant de charge essentiellement constant, lorsque la tension de suivi est essentiellement égale à une tension de seuil,
dans lequel le deuxième courant de charge essentiellement constant est inférieur au premier courant de charge essentiellement constant de 10 à 22 % du premier courant de charge essentiellement constant,
et dans lequel le premier courant de charge essentiellement constant est d'au moins 20 mA et d'au plus 250 mA.

9. Système de charge selon la revendication 8, le système de charge comprenant en outre un circuit intégré (IC) de charge et un dispositif de commande connecté de manière fonctionnelle à l'IC, dans lequel le dispositif de commande est configuré pour
charger la batterie avec un troisième, un quatrième et/ou un n-ième courant de charge essentiellement constant, dans lequel n représente un entier supérieur à quatre ;
commuter du deuxième au troisième, du troisième au quatrième et/ou du quatrième au n-ième courant de charge essentiellement constant, lorsque la tension suivie est essentiellement égale à une tension de seuil, de préférence une tension de seuil supérieur.

10. Dispositif médical implantable, ou IMD, comprenant un système de charge selon l'une quelconque des revendications 8 ou 9.

11. Programme informatique comprenant des instructions qui, lorsque le programme est exécuté par un ordinateur, amènent l'ordinateur à mettre en œuvre les étapes du procédé selon les revendications 1 à 7.
